# EUROPEAN PATENT SPECIFICATION

(11) **EP 3 952 930 B1**
(45) Date of publication and mention of the grant of the patent: **26.07.2023**
(21) Application number: 20721305.9
(22) Date of filing: 02.04.2020
(51) Int. Cl.: A61L 2/22, A61L 2/20

(54) **VAPORIZER OF DECONTAMINATING AGENTS**
VERDAMPFER VON DEKONTAMINATIONSMITTELN
VAPORISATEUR D'AGENTS DE DÉCONTAMINATION

(30) Priority: 08.04.2019 IT 201900005314
(43) Date of publication of application: 16.02.2022
(73) Proprietor: Tema Sinergie S.P.A., 48018 Faenza (RA) (IT)
(72) Inventor: PIANCASTELLI, Stefano, 48013 Brisighella (RA) (IT)
(74) Representative: Nesti, Antonio
(86) International application number: PCT/IB2020/053149
(87) International publication number: WO 2020/208484

(56) References cited:
- EP-A1- 1 142 592
- EP-B1- 1 142 592
- WO-A1-91/17804
- US-A1- 2004 237 466
- US-A1- 2011 203 914

## Description

### Technical Field of the Invention

The invention relates to a vaporizer of decontaminating agents, and in particular to a hydrogen peroxide vaporizer for sterilization plants, for example of environments or containers for sanitary or industrial use.

### Background art

At present, devices are known on the market for the generation of hydrogen peroxide, in a highly concentrated vapour state, for decontaminating sealed chambers such as: Isolators, airlocks, preparation-passers or handling cells.

In fact, hydrogen peroxide (H2O2) is known for its broad-spectrum decontamination power and its use as a decontaminating agent is now a standard in various industries, especially the food and pharmaceutical industries. The purpose of these devices is therefore to generate an airflow loaded with hydrogen peroxide which can be conveyed to the environment to be decontaminated.

In particular, for example from EP1425048, vaporizers are known comprising a metal mass with high thermal capacity which provide an airflow branch of pressurized air and an injection branch of the peroxide which meet inside the vaporizer so that the peroxide is conveyed by the air on the hot walls, causing their vaporization before being extracted by an outlet nozzle and used as a decontaminating agent.

Generally the peroxide in liquid form is introduced into the airflow through suitable drippers, fed by peristaltic pumps, consequently with a fairly coarse control of both the diameter of the drop that must vaporize and the peroxide flow (g/min).

US2004/237466 discloses a vaporizer in which an injection nozzle is used which is large enough to generate a solid/liquid jet with a diameter of a few tenths of a millimetre without the risk of clogging. US2011203914 discloses a vaporization device provided with an inlet configured to introduce the flow of vector fluidtoward a hot wall of a vaporization chamber having a cyclonic geometry.

However, these systems have some drawbacks, in particular in relation to the efficiency of the vaporization with respect to the energy used.

### Object of the Invention

A first object of the invention is to propose a vaporizer, as disclosed in the appended claims, free from the above-mentioned drawbacks which guarantees a high vaporization efficiency of the decontaminating agent introduced into the vaporizer.

### Summary of the Invention

These and further technical aims and advantages have been achieved according to the invention with a device according to at least one of the attached claims.

A first advantage obtained according to the invention consists essentially in the cyclonic form of the vaporization chamber, which by forcing the air to make spiral trajectories pushes the micro-drops of peroxide onto the hot walls of the vaporizer by centrifugal force, guaranteeing the immediate and complete vaporization of the injected liquid.

The cyclonic shape also allows the vaporization chamber to be realized in a thin wall, i.e. with a high ratio between the volume of the vaporization chamber and the total volume (chamber volume + heated mass), all while maintaining geometries that couple well with easily available heating systems. The thin wall of the vaporizer considerably lowers its thermal capacity, allowing a much more precise control of the vaporization temperature and an optimization of the power used for vaporization with the same peroxide flow.

### List of Drawings

These and other advantages will be better understood by anyone skilled in the art from the description below and the accompanying drawings, given as a nonlimiting example, wherein:

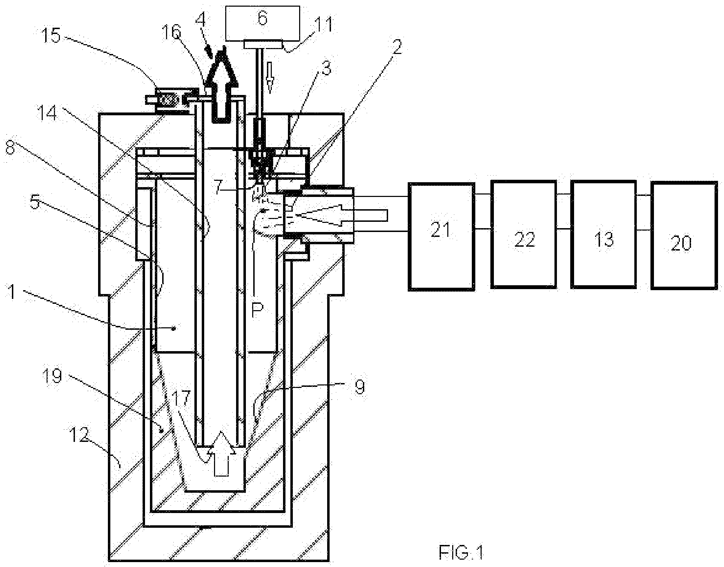

- fig.1 shows a vaporizer according to the invention;
- figures 2, 2a, 2b, 2c respectively show a side view, in the section A-A, from above, and a perspective view of a vaporization chamber according to the invention.

### Detailed Description

With reference to the attached drawings, a vaporizer of decontaminating agents, preferably oxygen peroxide, is described.

The vaporizer comprising a vaporization chamber 1 with hot walls 5 with external insulation 12, into which a first inlet 2 of a flow of a vector fluid, preferably dry, hot air driven by a fan 13 with a controlled, dehumidified, filtered and heated flow rate by suitable means 20, 22, 21 arranged upstream of the inlet 2 into the chamber 1.

By way of example, the preheating means 21 can be constituted by suitable resistances inserted inside the air ducts; the dehumidification means, by way of example, can be via a refrigeration cycle or a silica gel, the latter with rotor or static technologies.

Preferably, the temperature of the air entering the chamber 1 is about 55 - 65 °C, controlled for example via thermocouple, while the temperature of the vapour flow leaving the vaporizer is about 70 - 80 °C, measured for example by a thermocouple, but not necessarily controlled.

The flow of vector fluid is directed towards the hot wall 5 in a tangential direction thereto, and encounters inside the chamber 1 a flow of a liquid decontaminating agent, for example a 35% solution of oxygen peroxide, coming from a second inlet 3 and interfering with the flow of vector fluid at a meeting point P, for example being injected transversely thereto, to be dragged and vaporized via thermal flash in contact with the hot wall 5.

Preferably, the pressure in the injection inlet 3 of the decontaminating agent is controlled by means of a pressure switch located on the injection line, in order to avoid injecting into a clogged nozzle. By way of example, the pressure switch threshold can be set around 6 bar.

The chamber 1 further comprises an outlet 4 of the flow resulting from the meeting between the vaporized decontaminating agent and the transport vector fluid.

According to the invention, the vaporizer comprises injection means 6 arranged upstream of the second inlet 3 and associated therewith to inject an atomized flow of micro-drops of decontaminating agent.

Preferably, the injection means comprise a pump 6, for example a volumetric pump, equipped with an encoder 11 to feed the second inlet 3 with a controlled flow of liquid decontaminating agent equal to about 5.0 - 50.0 g/min, preferably 5.5- 30.0 g/min at a pressure between 1 and 4 bar, preferably 2-4 bar, established according to the injection rate of the decontaminating agent, a higher rate requiring a higher pressure at the injection nozzle 7.

In the various possible operating conditions, the nozzle 7 can be equipped with calibrated injection holes in the chamber 1 with an average diameter comprised between 0.05 and 0.25 mm, preferably 0.22 mm.

Equivalently, the peroxide injection circuit can consist of a pressurized tank with an ON/OFF dosing valve: the pressure of the pressurized tank containing liquid peroxide can be maintained by compressed air or, if containing only liquid, by means of an electronically controlled diaphragm pump or gear pump.

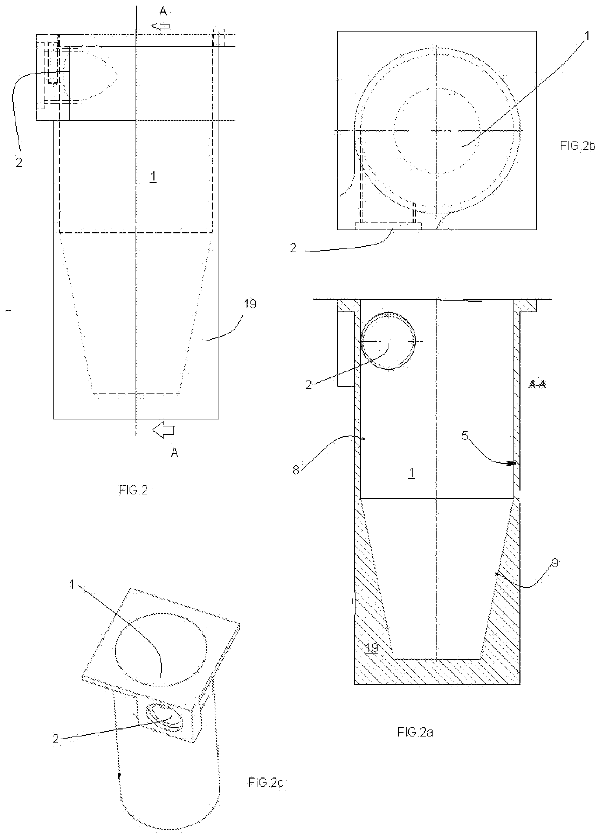

With reference in particular to figures 2, 2a-2c, the vaporization chamber 1 is obtained in a hollow metal body 19 so as to obtain a ratio between the volume of the vaporization chamber and the total volume given by the sum of the volume of the chamber 1 and of the heated body 19 greater than 55%.

This feature allows obtaining high energy efficiency and reducing thermal inertia.

Furthermore, the geometry of the chamber 1 is such as to present a ratio between the vaporization surface defined by the hot wall in contact with the decontaminating agent and the vacuum volume, i.e. the volume of the vaporization chamber greater than 0.4 cm-1.

This feature allows obtaining high productivity and reducing the quantity per surface unit of the residue that forms on the hot surface upon the vaporization by contact, and which tends to reduce the efficiency of the vaporizer and require cleaning and maintenance.

Preferably the chamber 1 has a cyclone geometry comprising a first portion 8 of a hot wall near the inlet 2 formed by a cylindrical section wall, for example made of metal, preferably aluminium, and a second conical portion 9 of hot wall near the outlet 4, converging in the direction of the vapour flow.

Advantageously, the tangential introduction of the airflow and the geometry of the vaporization chamber determine a spiral trajectory of the flow of atomized decontaminating agent which is transported by the vector fluid from the meeting point P towards the hot wall 5 and therefore, by centrifugal force, is kept in contact with the first portion 8, vaporized by thermal flash, and induced to the outlet 4 due to the convergent conical geometry of the second portion 9 of the chamber 5.

In the illustrated embodiment example, the outlet 4 is equipped with a temperature sensor 15 of the vapour produced and comprises a duct 14 arranged inside the chamber 1 and extended from an internal lower point 17 near the convergent end of the conical portion 9 to an external upper point of extraction of the vaporized decontaminating agent 16.

In an operating example, the airflow generated by the fan 13 is previously dehumidified by a dryer 20 and the air entering the chamber 1, possibly filtered by a filter 22, is pre-heated by a heating unit 21, so as to enter the vaporizer hot and dry and therefore ready to support vapour.

The hot, dry airflow is then conveyed into the vaporizer, where the meeting point P is located between the airflow and the injection flow, transversal to each other.

When the step pump 6 pushes the solution of decontaminating agent, in the example described oxygen peroxide, towards the vaporizer, the small size of the nozzle 7 and the ability of the pump 6 to generate pressure result in an atomization of the liquid which therefore splits into micro-drops passing through the nozzle.

These micro-drops collide with the airflow in the vaporizer. The cyclonic shape of the vaporizer thus obliges the flow of vector fluid that enters tangent to the upper surface of the inside of the vaporizer and the micro-drops dragged by the vector fluid to follow spiral trajectories, while the centrifugal force pushes the micro-drops towards the hot walls of the vaporization chamber.

The result is a thermal flash that brings the peroxide solution to the vapour state. The airflow will thus be charged with peroxide vapour and will be conveyed and pushed by the prevalence of the fan to the bottom of the vaporizer, at the portion 9 of the hot wall, towards the outlet 4 and from there to the environment or the container by means of one or more diffuser nozzles.

## Claims

1. A vaporizer of decontaminating agents, preferably oxygen peroxide, comprising
a vaporization chamber (1) with hot walls (5),
a first inlet (2) in said chamber (1) of a flow of a vector fluid, preferably air, directed towards the hot wall (5),
a second inlet (3) in said chamber (1) comprising injection means (6) configured to inject an atomized flow of micro-drops of decontaminating agent, so that said decontaminating agent is interfering in a meeting point (P) with said flow of vector fluid and is be dragged and vaporized in contact with the hot wall (5),
an outlet (4) from the chamber (1) of a flow of vaporized decontaminating agent transported by said vector fluid, said inlet (2) being configured to introduce the flow of vector fluid in a tangential direction to the hot wall (5) and said vaporization chamber (1) has a cyclone geometry with a first portion (8) in a proximal position with respect to said first inlet (2) and a second portion (9) with a conical geometry converging to the bottom of said vaporization chamber in order to induce a spiral trajectory of said flow of vaporized decontaminating agent carried by said vector fluid between said meeting point (P) and said outlet (4)
**characterized in that** the outlet (4) comprises a duct (14) arranged inside the chamber (1) and extended from an internal inlet point (17) proximal to the bottom of said chamber to an external extraction point (16) remote from the bottom of said vaporization chamber.

2. The vaporizer according to claim 1, wherein said injection means comprise a pump (6) for feeding a flow of liquid decontaminating agent at a pressure of between 1 and 4 bar and at least one nozzle (7) with calibrated injection holes in the chamber (1) with an average diameter comprised between 0.05 and 0.25 mm.

3. The vaporizer according to claim 2, wherein said injection means are configured to inject from 5.5 g/min to 30 g/min of liquid decontaminating agent at a pressure comprised between 2 and 4 bar increasing with the growth of said pressurized flow of liquid decontaminating agent.

4. The vaporizer according to claim 2, wherein said pump (6) is a volumetric pump with encoder (11).

5. The vaporizer according to one of the preceding claims, wherein said vaporization chamber (1) is obtained in a hollow metal body (19) with a ratio between the volume of the vaporization chamber and the total volume given by the sum of the volume of the chamber (1) and of the heated body (19) greater than 55%.

6. The vaporizer according to one of the preceding claims, the ratio between the vaporization surface defined by the hot wall (5) in contact with the decontaminating agent and the volume of the vaporization chamber (1) is greater than 0.4 cm-1.

7. The vaporizer according to one of the preceding claims, wherein said vaporization chamber is metallic, preferably in aluminium.

8. The vaporizer according to one of the preceding claims, comprising means (21) for pre-heating said flow of vector fluid upstream of said inlet (2).

9. The vaporizer according to one of the preceding claims, wherein the outlet (4) is equipped with a temperature sensor (15) of the vapour produced.

10. The vaporizer according to one of the preceding claims, comprising an external insulation (12) of said vaporization chamber (1).

## Patentansprüche

1. Verdampfer von Dekontaminationsmitteln, vorzugsweise Sauerstoffperoxid, umfassend
eine Verdampfungskammer (1) mit heißen Wänden (5),
einen ersten Einlass (2) in der Kammer (1) für einen Strom eines Vektorfluids, vorzugsweise Luft, der auf die heiße Wand (5) gerichtet ist,
einen zweiten Einlass (3) in der Kammer (1) umfassend Injektionsmittel (6), die konfiguriert sind, um einen zerstäubten Strom von Mikrotropfen eines Dekontaminationsmittels zu injizieren, derart, dass das Dekontaminationsmittel an einem Treffpunkt (P) mit dem Vektorfluidstrom interferiert und in Kontakt mit der heißen Wand (5) gezogen und verdampft wird,
einen Auslass (4) aus der Kammer (1) eines Stroms von verdampftem Dekontaminationsmittel, der von dem Vektorfluid transportiert wird,
wobei der Einlass (2) konfiguriert ist, um den Vektorfluidstrom in einer tangentialen Richtung zu der heißen Wand (5) einzuleiten, und die Verdampfungskammer (1) eine Zyklongeometrie mit einem ersten Abschnitt (8) in einer proximalen Position in Bezug auf den ersten Einlass (2) und einem zweiten Abschnitt (9) mit konischer Geometrie aufweist, die zum Boden der Verdampfungskammer konvergieren, um eine spiralförmige Bahn des von dem Vektorfluid getragenen Stroms an verdampften Dekontaminationsmittel zwischen dem Treffpunkt (P) und dem Auslass (4) einzuführen,
**dadurch gekennzeichnet, dass** der Auslass (4) einen Kanal (14) umfasst, der innerhalb der Kammer (1) angeordnet ist und sich von einer internen Einlassstelle (17) proximal zum Boden der Kammer zu einer externen Entnahmestelle (16) entfernt vom Boden der Verdampfungskammer erstreckt.

2. Verdampfer nach Anspruch 1, wobei die Injektionsmittel eine Pumpe (6) zum Zuführen eines Stroms von flüssigem Dekontaminationsmittel bei einem Druck zwischen 1 und 4 bar und mindestens eine Düse (7) mit kalibrierten Injektionslöchern in der Kammer (1) mit einem durchschnittlichen Durchmesser zwischen 0,05 und 0,25 mm umfassen.

3. Verdampfer nach Anspruch 2, wobei die Injektionsmittel konfiguriert sind, um 5,5 g/min bis 30 g/min flüssiges Dekontaminationsmittel bei einem Druck zwischen 2 und 4 bar, der mit der Zunahme des unter Druck stehenden Stroms von flüssigem Dekontaminationsmittel zunimmt, zu injizieren.

4. Verdampfer nach Anspruch 2, wobei es sich bei der Pumpe (6) um eine volumetrische Pumpe mit Codierer (11) handelt.

5. Verdampfer nach einem der vorhergehenden Ansprüche, wobei die Verdampfungskammer (1) in einem hohlen Metallkörper (19) erhalten wird, wobei ein Verhältnis zwischen dem Volumen der Verdampfungskammer und dem Gesamtvolumen, gegeben durch die Summe des Volumens der Kammer (1) und des beheizten Körpers (19), größer als 55 % ist.

6. Verdampfer nach einem der vorhergehenden Ansprüche, wobei das Verhältnis zwischen der Verdampfungsfläche, die durch die mit dem Dekontaminationsmittel in Kontakt stehende heiße Wand (5) definiert ist, und dem Volumen der Verdampfungskammer (1) größer als 0,4 cm-1 ist.

7. Verdampfer nach einem der vorhergehenden Ansprüche, wobei die Verdampfungskammer metallisch ist, vorzugsweise aus Aluminium.

8. Verdampfer nach einem der vorangehenden Ansprüche, umfassend Mittel (21) zum Vorheizen des Vektorfluidstroms stromaufwärts von dem Einlass (2).

9. Verdampfer nach einem der vorhergehenden Ansprüche, wobei der Auslass (4) mit einem Temperatursensor (15) des erzeugten Dampfes ausgestattet ist.

10. Verdampfer nach einem der vorhergehenden Ansprüche, umfassend eine Außenisolierung (12) der Verdampfungskammer (1).

## Revendications

1. Vaporisateur d'agents de décontamination, de préférence du peroxyde d'oxygène, comprenant une chambre de vaporisation (1) à parois chaudes (5),
une première entrée (2) dans ladite chambre (1) d'un flux d'un fluide vecteur, de préférence de l'air, dirigé vers la paroi chaude (5),
une deuxième entrée (3) dans ladite chambre (1) comprenant des moyens d'injection (6) configurés pour injecter un flux atomisé de microgouttes d'agent de décontamination, de telle sorte que ledit agent de décontamination interfère en un point de rencontre (P) avec ledit flux de fluide vecteur et est traîné et vaporisé en contact avec la paroi chaude (5),
une sortie (4) de la chambre (1) d'un flux d'agent de décontamination vaporisé transporté par ledit fluide vecteur,
ladite entrée (2) étant configurée pour introduire le flux de fluide vecteur dans une direction tangentielle à la paroi chaude (5) et ladite chambre de vaporisation (1) a une géométrie cyclonique avec une première portion (8) dans une position proximale par rapport à ladite première entrée (2) et une deuxième portion (9) avec une géométrie conique convergeant vers le fond de ladite chambre de vaporisation afin d'induire une trajectoire en spirale dudit flux d'agent de décontamination vaporisé porté par ledit fluide vecteur entre ledit point de rencontre (P) et ladite sortie (4)
**caractérisé en ce que** la sortie (4) comprend un conduit (14) agencé à l'intérieur de la chambre (1) et étendu d'un point d'entrée interne (17) proximal au fond de ladite chambre à un point d'extraction externe (16) éloigné du fond de ladite chambre de vaporisation.

2. Vaporisateur selon la revendication 1, dans lequel lesdits moyens d'injection comprennent une pompe (6) pour apporter un flux d'agent de décontamination liquide à une pression entre 1 et 4 bars et au moins une buse (7) avec des trous d'injection calibrés dans la chambre (1) avec un diamètre moyen compris entre 0,05 et 0,25 mm.

3. Vaporisateur selon la revendication 2, dans lequel lesdits moyens d'injection sont configurés pour injecter de 5,5 g/min à 30 g/min d'agent de décontamination liquide à une pression comprise entre 2 et 4 bars augmentant avec la croissance dudit flux pressurisé d'agent de décontamination liquide.

4. Vaporisateur selon la revendication 2, dans lequel ladite pompe (6) est une pompe volumétrique avec un encodeur (11).

5. Vaporisateur selon l'une des revendications précédentes, dans lequel ladite chambre de vaporisation (1) est obtenue dans un corps (19) en métal creux avec un rapport entre le volume de la chambre de vaporisation et le volume total donné par la somme du volume de la chambre (1) et du corps (19) chauffé supérieur à 55 %.

6. Vaporisateur selon l'une des revendications précédentes, le rapport entre la surface de vaporisation définie par la paroi chaude (5) en contact avec l'agent de décontamination et le volume de la chambre de vaporisation (1) est supérieur à 0,4 cm⁻¹.

7. Vaporisateur selon l'une des revendications précédentes, dans lequel ladite chambre de vaporisation est métallique, de préférence en aluminium.

8. Vaporisateur selon l'une des revendications précédentes, comprenant des moyens (21) de préchauffage dudit flux de fluide vecteur en amont de ladite entrée (2).

9. Vaporisateur selon l'une des revendications précédentes, dans lequel la sortie (4) est équipée d'un capteur de température (15) de la vapeur produite.

10. Vaporisateur selon l'une des revendications précédentes, comprenant une isolation externe (12) de ladite chambre de vaporisation (1).
